# EUROPEAN PATENT SPECIFICATION

(11) **EP 1 813 235 B1**
(45) Date of publication and mention of the grant of the patent: **22.02.2012**
(21) Application number: 07100519.3
(22) Date of filing: 15.01.2007
(51) Int. Cl.: A61F 13/15

(54) **Disposable body fluid absorbent wearing article**
Körperflüssigkeiten absorbierender Einweg-Bekleidungsartikel
Article vestimentaire jetable absorbant pour fluides anatomiques

(30) Priority: 27.01.2006 JP 2006019712; 26.10.2006 JP 2006291652
(43) Date of publication of application: 01.08.2007
(73) Proprietor: UNI-CHARM CORPORATION, Shikokuchuo-shi, Ehime 799-0111 (JP)
(72) Inventor: Sakakibara, Akiko, Kagawa-ken Kagawa (JP); Wada, Mitsuhiro, Kagawa-ken Kagawa (JP)
(74) Representative: Fitchett, Stuart Paul

(56) References cited:
- EP-A- 1 479 361
- US-A- 4 790 840
- US-A1- 2001 021 833
- US-B1- 6 265 631

## Description

The present invention relates generally to a disposable body fluid absorbent wearing article such as a disposable sanitary napkin or disposable diaper and particularly to such a wearing article adapted to prevent itching from occurring in the course of using such a wearing article.

It is also well known that an environment defined between the article wearer's skin and the wearing article becomes abnormal as the article is worn and causes the wearer to experience various types of discomfort such as itching. To improve such an abnormal environment, US Patent Application Publication Number 2001/0021833 and Unexamined Japanese Patent Application Number 2003-522600 disclose wearing articles provided with thermal cell actuator adapted for heat transfer to, or for heat removal from, at least a portion of the body fluid absorbent wearing article. The thermal cell actuator adapted for heat remove is also referred to as a cooling device and, regarding this, a phase change material containing therein volatile liquid serving to lower the temperature under the effect of evaporation cooling is described in JP 2003-522600 as an example of the cooling device.

While JP 2003-522600 exemplarily describes incorporation of the phase change material in the diaper, none of methods for keeping such a phase change material usable for a long period elapsing from production of the diaper to actual use of this diaper.

It is an object of the present invention to improve the disposable body fluid absorbent wearing article including a coolant so that the coolant can be kept usable for a long period so as to improve the shelf life of the article.

According to the present invention, there is provided a disposable body fluid absorbent wearing article comprising a liquid-pervious topsheet, a liquid-impervious backsheet, a body fluid absorbent core sandwiched between these two sheets and a coolant, wherein: said coolant is a water bearing gelatinous sheet adapted to exercise a cooling function as said water begins to evaporate, characterised in that
(i) said coolant is interposed between said topsheet and said core, or between said core and said backsheet, said backsheet being air-impervious, and in said coolant is enclosed by an air-impervious sheet covering a surface of said topsheet facing away from said core and detachably bonded to this surface and said backsheet, or
(ii) said coolant is provided on an outer surface of said backsheet facing away from said core, said backsheet having air-impervious nature, and in a manner that said coolant is enclosed by an air-impervious sheet prepared separately of said backsheet and detachably bonded to said outer surface of said backsheet and said backsheet
and the air-impervious sheet is torn off immediately before the article is actually used.

According to further another preferred embodiment of the present invention, the coolant has an extent corresponding to at least 9% of the surface area of the core on its side facing the topsheet or the surface area of the core on its side facing the backsheet.

The wearing article according to the present invention includes the coolant comprising water bearing gelatinous sheet adapted to exercise a cooling function as water begins to evaporate and is wrapped with an air-impervious sheet during a period on the shelf. The coolant can maintain its cooling capacity unless the air-impervious sheet is torn off immediately before the article is actually used because the air-impervious sheet prevents water contained in the coolant from evaporating.

In the embodiment of the invention wherein the backsheet is air-impervious and the coolant is attached to the outer surface of this backsheet in such a manner that the coolant is enclosed by the backsheet and the air-impervious sheet detachably bonded to the backsheet, the backsheet is partially utilized to enclose the coolant and thereby a cost of packaging material for the coolant can be reduced.

In the embodiment of the invention wherein the coolant is interposed between the topsheet and the core or between the core and the backsheet, and enclosed by the air-impervious sheet covering the topsheet and detachably bonded thereto and the air-impervious backsheet, the backsheet is partially utilized to enclose the coolant and thereby a cost of packaging material for the coolant can be reduced.

In the embodiment of the invention wherein the coolant has an extent corresponding to at least 9% of the surface area of the core on its side facing the topsheet or the surface area of the core on its side facing the backsheet, the anti-itch effect is reliably achieved during use of the wearing article according to the invention.
Fig. 1 is a partially cutaway perspective view showing a menstruation pad;
Fig. 2 is a sectional view taken along line II-II in Fig. 1;
Fig. 3 is a sectional view schematically illustrating an apparatus used to measure an amount of permeating water vapor;
Fig. 4 is a graph diagram plotting a measurement result for amount of permeating water vapor;
Fig. 5 is a graph diagram plotting a change in temperature and humidity within an undergarment put on the wearer's body;
Fig. 6 is a view similar to Fig. 2, showing one preferred embodiment of the invention;
Fig. 7 is a view similar to Fig. 2, showing another preferred embodiment of the invention; and
Fig. 8 is a partially cutaway plan view showing an embodiment of the invention.

Details of the body fluid absorbent wearing article according to the present invention will be more fully understood from the description of a sanitary napkin as embodiments of the invention with reference to the accompanying drawings.

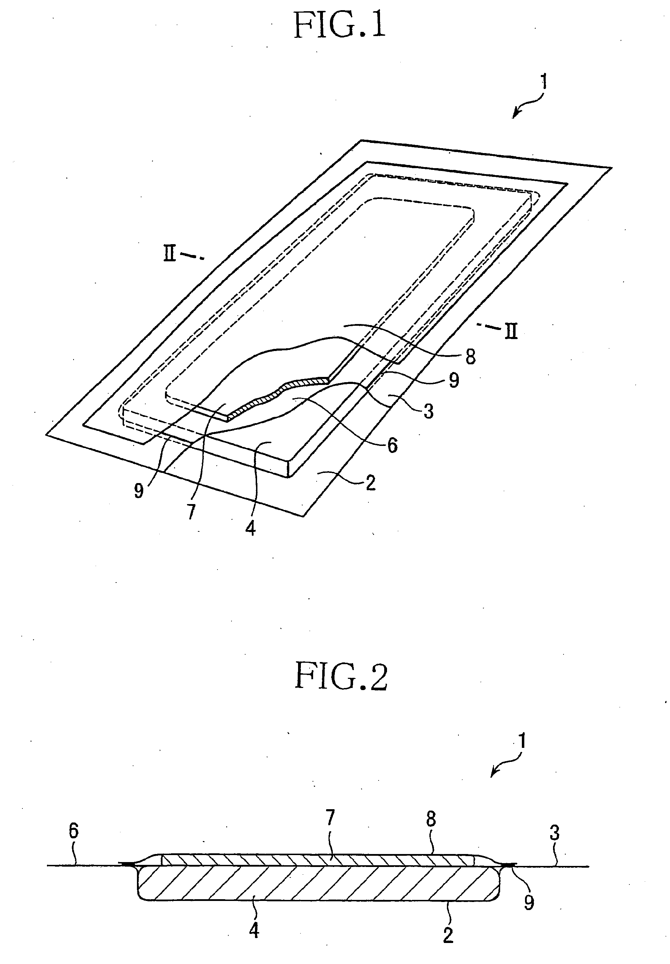

Fig. 1 is a partially cutaway perspective view showing a menstruation pad 1 and Fig. 2 is a sectional view taken along the line II-II in Fig. 1. The menstruation pad 1 comprises a liquid-pervious topsheet 2, a liquid-impervious backsheet 3 and a body fluid absorbent core 4 sandwiched between these two sheets 2, 3. The menstruation pad 1 further comprises a coolant 7 layered on an outer surface 6 of the backsheet 3 facing away from the core 4 and an air-impervious cover sheet 8 covering the coolant 7 from above as viewed in Figs. 1 and 2. The topsheet 2 and the backsheet 3 extend outward beyond a peripheral edge of the core 4 so as to be put flat and bonded together around the core 4 by an appropriate adhesive or sealing technique. The coolant 7 is a sheet strip made of a gelatinous material which is attached to the outer surface 6 of the backsheet 3 in a manner that the coolant 7 covers at least a portion of the core 4 by the intermediary of the backsheet 3. The cover sheet 8 extends outward beyond a peripheral edge of the coolant 7 so as to be put flat together with the backsheet 3 and detachably bonded to the outer surface 6 of the backsheet 3 by means of a pressure-sensitive adhesive 9. The menstruation pad 1 constructed as has been described above may be worn by the following steps of peeling off the cover sheet 8 from the back sheet 3 to expose the coolant 7, attaching the coolant 7 to an undergarment (not shown) on the inner surface of a crotch region thereof so that the coolant 7 may serve as a fastener means or slip stopper means, and wearing the menstruation pad 1 together with the undergarment.

In such menstruation pad 1, the topsheet 2 is formed from a liquid-pervious sheet selected from the group including a nonwoven fabric of melt blown- or spun lace type and a perforated plastic film. The backsheet 3 is formed from a plastic film. The core 4 is formed from fluff pulp fibers or a mixture of fluff pulp fibers and super-absorbent polymer particles. The coolant 7 is formed from water bearing endothermic materials functioning to cool the area surrounding the coolant 7 as an amount of water contained therein evaporates under the effect of evaporative heat. An example of such coolant 7 is a gelatinous material comprising polyacrylic acids, water, N-vinyl acetamide polymers, and polyvalent metals. It should be understood that the term "polyacrylic acids" used herein refers to, in addition to polyacrylic acids, metallic salts thereof such as sodium polyacrylates and ammonium salts thereof. The term "polyvalent metals" used herein should be understood to include, in addition to aluminum, magnesium and calcium, hydroxides of these metals. Such gelatinous material may be used in a manner that the outer surface 6 of the backsheet 3 may be coated with this gelatinous material to form a layer or a sheet having a thickness in a range of 0.5 to 5 mm and a basis weight in a range of 600 to 3200 g/m² or a carrier sheet made of a nonwoven fabric or a plastic film is coated with this gelatinous material to form a composite sheet strip which is, in turn, bonded to the outer surface 6 of the backsheet 3. A specific example of the gelatinous material comprises about 5 weight % of polyacrylic acid, about 93 weight % of water, about 2 weight % of N-vinyl acetamide polymer and about 0.2 weight % of aluminum hydroxide. This specific gelatinous material has an endothermic ability based on a water evaporation rate in range of 55 to 80 g/m²/h at a normal temperature. This gelatinous material has a sufficient stickiness allowing such coolant sheet or layer to be attached to the backsheet 3 without an anxiety that this coolant sheet or layer might be unintentionally detached from the backsheet 3. The stickiness of this gelatinous material further allows the coolant 7 previously formed in a sheet or a layer to be pressure bonded to the backsheet 3. When the gelatinous material is used in the form of the composite sheet strip, the stickiness of the gelatinous material may be utilized to pressure-bond this composite sheet strip to the backsheet 3 or an adhesive or a pressure-sensitive adhesive may be utilized to bond the carrier sheet strip constituting the composite sheet strip to the backsheet 3. If the coolant 7 is attached to the backsheet 3 with the gelatinous material of the coolant 7 facing the inner surface of the undergarment, the gelatinous material will at least temporarily serve as a fastener means or a slip stopper means for the menstruation pad 1 with respect to the undergarment. The cover sheet 8 is formed from an air-impervious plastic film which is integrated with the air-impervious backsheet 3 so as to wrap the coolant 7 in a hermetically sealing fashion and thereby to prevent water contained in the coolant 7 from evaporating prior to actual use of the menstruation pad 1.

The inventors found that, while an ambient temperature in a space defined between the skin of the wearer's crotch zone and the undergarment is usually about 34°C when the undergarment is worn, the ambient temperature rises to about 36°C and a relative humidity readily exceeds 90% when the menstruation pad is worn in combination with the undergarment and an excretion amount of menstrual fluid is relatively great. Even when the excretion amount of the menstrual fluid is relatively small, the ambient temperature rises to about 35°C and the relative humidity readily exceeds 90%. At the ambient temperature of 35 to 36°C and the relative humidity exceeding 90%, a rate of itch occurrence for the wearer is substantially higher than at the ambient temperature of about 34°C. The menstruation pad 1 including the coolant 7 as shown by Fig. 1 effectively alleviates this problem. Specifically, the menstruation pad 1 may be attached to the undergarment after the cover sheet 8 has been peeled off therefrom and then may be worn together with the undergarment to ensure that water vapor within the undergarment is condensed in the vicinity of the coolant 7 under the cooling effect caused as the amount of water contained in the coolant 7 evaporates and a gradient of the water vapor partial pressure is generated between the wearer's skin and the coolant 7. Thereupon the water vapor present in the vicinity of the wearer's skin migrates toward the coolant 7, resulting in drop of the humidity as well as the temperature in the vicinity of the wearer's skin. Consequently, the number of subjects wearing the menstruation pads according to the invention and experiencing itch was apt to be fewer than the number of subjects wearing the menstruation pads each not provided with any kind of coolant. These findings support the fact that the coolant 7 is effective to lower the ambient temperature between the menstruation pad 1 and skin of the subject wearing the menstruation pad 1 and to promote movement of water vapor from the wearer's skin toward the menstruation pad 1.

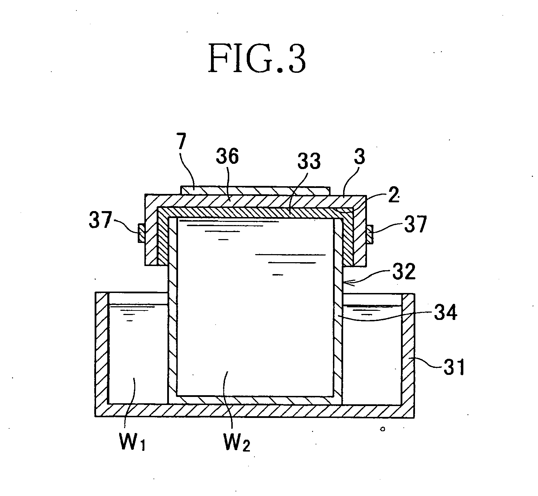

Fig. 3 is a schematic sectional view illustrating an apparatus used to determine a degree of water vapor migration under the effect of the coolant 7. This apparatus is adapted to be used in a room at a temperature of 34°C and a relative humidity of 60% and comprises a keep-warm bath 31 adapted to keep water W₁ filled therein at a temperature of 35°C and a cylindrical cup 32 having an inner diameter of 50 mm and a depth of 75 mm placed in the keep-warm bath 31 so that water W₂ filled in the cup 32 may be kept at a temperature of 35°C to be evaporated upward as viewed in Fig. 3. An opening of the cup 32 was covered with a microporous polytetrafluoroethylene film having a thickness of 25 µm and porosity of 80% (commercially available from Japan Goretex in the trade name of GORE-TEX) by tightly holding the film 33 in contact with a peripheral wall 34 of the cup 32. A test strip 36 of 70 mm x 70 mm cut from the menstruation pad was placed on the film 33 with the topsheet 2 facing the film 33. A peripheral portion of the test strip 36 was held in tight contact with the peripheral wall by the intermediary of the film 33. To determine the effect of the coolant 7 by using the test strip 36, the backsheet 3 defining the upper layer of the test strip 36 as viewed in Fig. 3 was coated with the coolant 7 to define a layer of the coolant 7 having a diameter in a range of 50 to 60 mm and a thickness of about 3 mm. The cup 32 including the test strip 36 attached thereto was measured in weight every one hour and a variation in the weight was divided by the opening area of the cup 32 to obtain a migration amount of water vapor in unit of g/m².

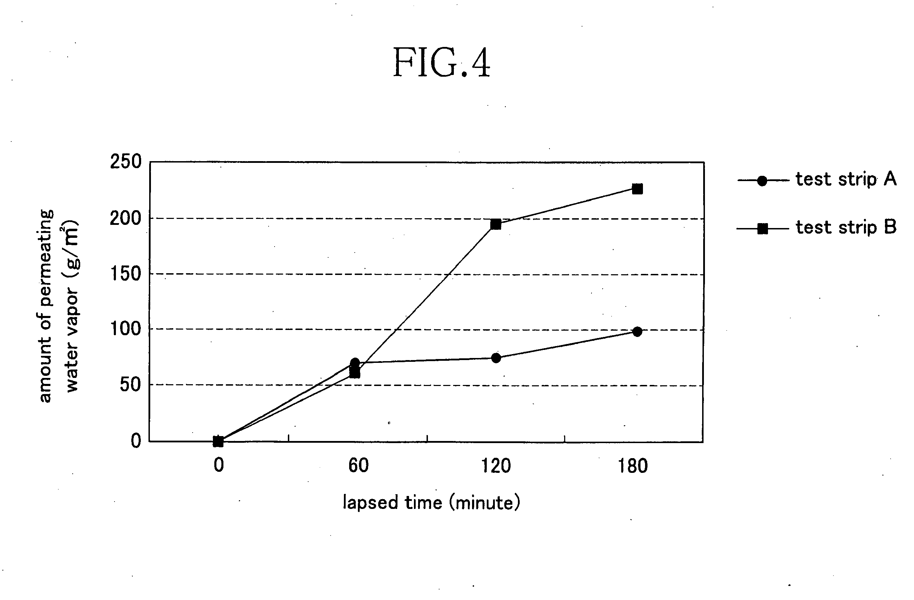

Fig. 4 is a graphic diagram plotting a measurement result for amount of permeating water vapor varying as time elapses. The apparatus of Fig. 3 was used to make this measurement on a test strip A prepared using the commercially available menstruation pad from Uni-Charm Corporation (the present applicant) in the trade name of SOFY (active slim instant guard) and a test strip B prepared by coating the backsheet of the test strip A with the coolant 7 comprising about 5 weight % of N-vinyl acetamide polymer and about 0.2 weight % of aluminum hydroxide to a thickness of 3 mm. As will be apparent from this graphic diagram, a differential amount of permeating water vapor between the test strip B utilizing the coolant 7 and the test strip A not utilizing the coolant gradually increases as the time elapses. Fig. 4 thus supports a significant effect of the coolant 7 to promote the water vapor migration.

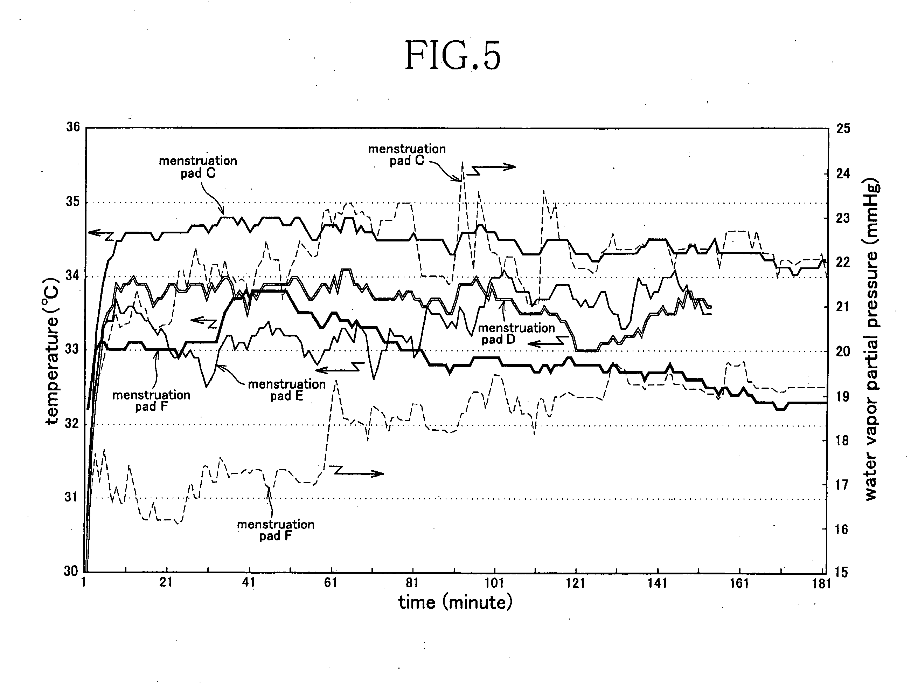

Fig. 5 indicates an observation result for test menstruation pads C, D, E and F worn by respective wearers. These test pads were prepared by coating the pads commercially available from Uni-Charm Corporation in the trade name of SOFY (active slim instant guard) on rear surfaces thereof with amounts of the coolant 7 as indicated by TABLE 1 below except the pad C. The respective test pads were worn by respective subjects together with the undergarment to observe a variation in temperature and humidity (water vapor partial pressure) within each of the undergarment, using the same coolant 7 as used in the observation of which the result is plotted in Fig. 4. Each of the respective test pads has the core shaped substantially in a rectangle dimensioned in 75 x 190 mm as viewed in the transverse direction and the longitudinal direction on both surfaces opposed to the topsheet and the backsheet, respectively. Each of the test pads is coated on the rear surface of the backsheet with the coolant 7 so as to be laid in the middle with respect to both the transverse direction and the longitudinal direction. The coolant 7 was coated substantially in a rectangular shape dimensioned to be uniformly 130 mm in the longitudinal direction and 3 mm in the thickness direction but to be varied in the transverse direction and thereby to vary the amount (the area ratio) of the coolant 7 with which one side of the core is coated. The temperature as well as the humidity was measured by means of a sensor (Thermo Recorder available from T & D in the trade name of ONDOTORI RHTR-72S) attached to each of the test pads C, D, E and F on the rear ends of the respective topsheets thereof. These test pads C, D, E and F were worn by one and same subject in a manner that the test pads C, D, E and F was worn in this order by the subject for the menstrual periods in every June. More specifically, during each of the menstrual periods, the test pad was replaced by the fresh one every three hours and the temperature as well as the humidity was measured 3 days after the respective menstrual periods had begun.

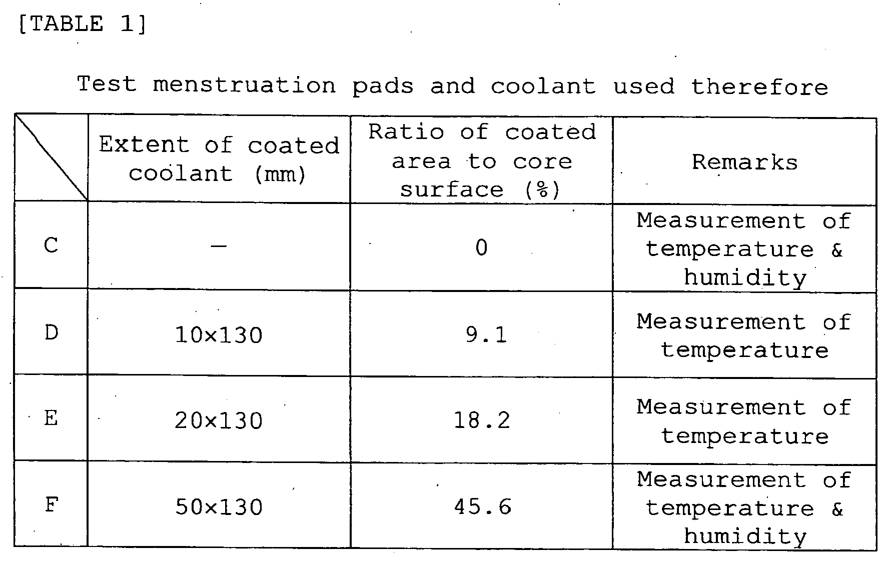

As will be apparent from Fig. 5, the temperature within a space defined between the topsheet of the test menstruation pad and the wearer's skin is apt to be gradually reduced as the area ratio at which the backsheet is coated with the coolant 7 is enlarged. From the measurement result for the test pad C on which the coolant coated area ratio is 0 and the test pad F on which the coolant coated area ratio is 45. 6%, it is obvious that use of the coolant 7 is apt to lower the humidity also in a manner similar to the case of the temperature.

TABLE 2 indicates the itch occurrence rate obtained by putting the same test menstruation pads C, D, E and F as those having been used for the measurement of which the result is indicated by Fig. 5 on 10 subjects, respectively. More specifically, for the respective test pads, percentages of the subjects who had reported to have experienced more or less itch occurrence until third days from the start of the menstrual period were indicated by TABLE 2 as the itch occurrence rate. As will be obvious from TABLE 2, the test pad D using the coolant 7 (at the coated area ratio: 9.1%) effectively reduced the itch occurrence rate. This effect is more significant in the case of the test pad E using the coolant 7 (at the coated area ratio: 18.2%) and further more significant in the case of the test pad F using the coolant 7 (at the coated area ratio: 45.6%).

**[TABLE 2]**

| Test menstruation pad | Itch occurrence rate (%) |
|---|---|
| C | 60.5 |
| D | 24.6 |
| E | 15.2 |
| F | 7.7 |

It is verified by the observation result plotted and indicated by Fig. 5 and TABLE 2, respectively, that the backsheet 3 of the menstruation pad 1 may be coated with the coolant 7 by an amount corresponding to at least 9% of the surface area of the core 4 on the side facing the backsheet 3 to alleviate itch and stuffiness possibly occurring in the course of wearing the pad 1 and this effect is further improved by using an amount of the coolant 7 corresponding to 18% or higher of the surface area of the core 4.

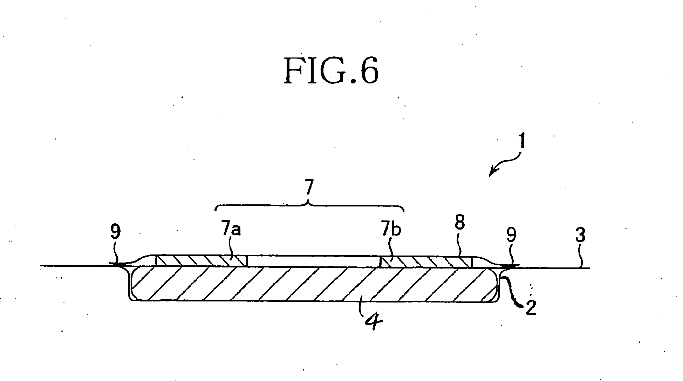

Fig. 6 is a view similar to Fig. 2, showing one preferred embodiment of the invention. The menstruation pad 1 according to this embodiment includes a pair of coolants 7a, 7b spaced from each other in the transverse direction of the pad 1 so that the backsheet 3 may be exposed upward as viewed in Fig. 6 between these two coolants 7a, 7b. The cover sheet 8 covers the coolants 7a, 7b and is detachably bonded to the backsheet 3 by the intermediary of the pressure-sensitive adhesive 9.

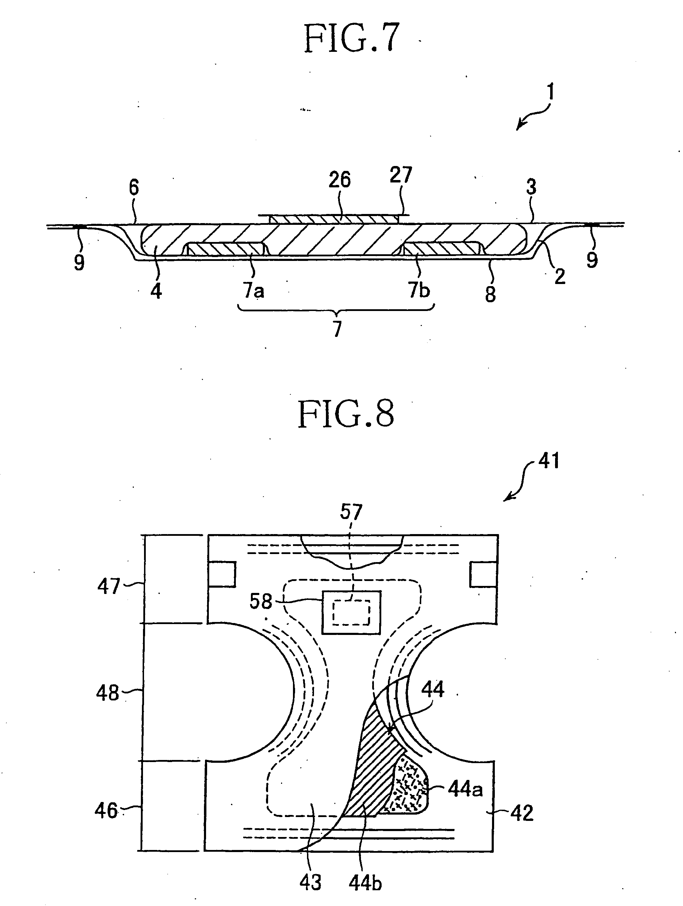

Fig. 7 also is a view similar to Fig. 2, showing another preferred embodiment of the invention. The menstruation pad 1 according to this embodiment includes the coolant 7 interposed between the topsheet 2 and the core 4 wherein the coolant 7 is divided into a pair of coolants 7a, 7b spaced from each other in the transverse direction of the pad 1. The coolants 7a, 7b are embedded, for the most part thereof in the thickness direction, in the core 4 so as to avoid inconvenient protrusion of these coolants 7a, 7b. While the backsheet 3 is liquid-impervious and air-impervious, the topsheet 2 is liquid-pervious and air-pervious. The topsheet 2 is covered with the air-impervious cover sheet 8 from below as viewed in Fig. 7. The cover sheet 8 is detachably bonded to the topsheet 2 by the pressure-sensitive adhesive 9. Because the coolant 7 is enclosed by the backsheet 3 and the cover sheet 8, the water contained in the coolant 7 cannot readily evaporate. The outer surface 6 of the backsheet 3 is coated with a pressure-sensitive adhesive 26 serving to fasten the pad 1 to the undergarment of the wearer and the pressure-sensitive adhesive 26 is covered with a release paper 27. In the case of this embodiment also, the coolant coated area ratio preferably corresponds to at least 9% of the surface area of the core 4 on the side facing the topsheet 2.

While the present invention has been described hereinabove with respect to the menstruation pad 1 as the typical embodiment thereof, the present invention is applicable to the other disposable wearing articles such as disposable diapers.

Fig. 8 is a partially cutaway plan view showing a disposable diaper 41 as an embodiment of the disposable bodily discharge absorbent wearing article according to the invention. The diaper 41 comprises a liquid-pervious topsheet 42, a liquid-impervious backsheet 43 and a body fluid absorbent core 44 sandwiched between these two sheets 42, 43. The backsheet 43 is not only liquid-impervious but also air-impervious and formed from a polyethylene film having a thickness of 30 µm. The core 44 comprises a water-absorbing material 44a such as fluff pulp fibers and super-absorptive polymer particles and a liquid-pervious sheet 44b such as a tissue paper. In the state as shown by Fig. 9, the diaper 41 is composed of, in a longitudinal direction, a front waist region 46, a rear waist region 47 and a crotch region 48 extending between these waist regions 46, 47. In the rear waist region 47, the backsheet 43 is coated on its outer surface with coolant 57 which is, in turn, covered with an air-impervious cover sheet 58. The cover sheet 58 extends outward beyond a peripheral edge of the coolant 57 and is detachably bonded to the outer surface of the backsheet 43 around the coolant 57. With the diaper 41 put on the wearer's body, the cover sheet 58 may be peeled off from the outer surface of the backsheet 43 to expose the coolant 57 and thereby to promote evaporation of the amount of water contained in the coolant 57. In such diaper 41, the presence of the coolant 57 can effectively prevent stuffiness and itch from occurring about the wearer's waist.

The present invention makes it possible to manufacture wearing articles including the coolant attached thereto with an improved shelf life, in other words, in such a manner in that the coolant does not readily evaporate.

## Claims

1. A disposable body fluid absorbent wearing article (1) comprising a liquid-pervious topsheet(2), a liquid-impervious backsheet (3), a body fluid absorbent core(4) sandwiched between these two sheets and a coolant(7), wherein:
said coolant is a water bearing gelatinous sheet adapted to exercise a cooling function as said water begins to evaporate, **characterised in that**
(i) said coolant (7) is interposed between said topsheet (2) and said core (4), or between said core (4) and said backsheet (3), said backsheet being air-impervious, and in said coolant is enclosed by an air-impervious sheet (8) covering a surface of said topsheet (2) facing away from said core and detachably bonded (9) to this surface and said backsheet (3), or
(ii) said coolant (7) is provided on an outer surface (6) of said backsheet (3) facing away from said core (4), said backsheet having air-impervious nature, and in a manner that said coolant (7) is enclosed by an air-impervious sheet (8) prepared separately of said backsheet and detachably bonded (9) to said outer surface (6) of said backsheet (3) and said backsheet (3)
and the air-impervious sheet is torn off immediately before the article is actually used.

2. The wearing article defined by Claim 1, wherein said coolant has an extent corresponding to at least 9% of the surface area of said core on its side facing said topsheet or the surface area of said core on its side facing said backsheet.

## Patentansprüche

1. Körperflüssigkeiten absorbierender Einweg-Bekleidungsartikel (1), umfassend eine flüssigkeitsdurchlässige Oberlage (2), eine flüssigkeitsundurchlässige rückseitige Lage (3), einen zwischen diesen beiden Lagen angeordneten Körperflüssigkeiten absorbierenden Kern (4) und ein Kühlmittel (7), wobei:
das besagte Kühlmittel eine wasserführende, gelatineartige Lage ist, die dazu ausgelegt ist, eine Kühlfunktion zur Wirkung zu bringen, wenn das besagte Wasser zu verdunsten beginnt, **dadurch gekennzeichnet, dass**
(i) das besagte Kühlmittel (7) zwischen der besagten Oberlage (2) und dem besagten Kern (4) oder zwischen dem besagten Kern (4) und der besagten rückseitigen Lage (3) angeordnet ist, wobei die besagte rückseitige Lage luftundurchlässig ist, und dass das besagte Kühlmittel von einer luftundurchlässigen Lage (8) umschlossen ist, die eine von dem besagten Kern abgekehrte Oberfläche der besagten Oberlage (2) bedeckt und lösbar mit dieser Oberfläche und der besagten rückseitigen Lage (3) verbunden (9) ist, oder dass
(ii) das besagte Kühlmittel (7) auf einer von dem besagten Kern (4) abgekehrten Außenfläche (6) der besagten rückseitigen Lage (3) angeordnet ist, wobei die besagte rückseitige Lage einen luftundurchlässigen Charakter hat, und zwar so, dass das besagte Kühlmittel (7) von einer luftundurchlässigen Lage (8) umschlossen ist, die separat von der besagten rückseitigen Lage hergestellt und lösbar mit der besagten Außenfläche (6) der besagten rückseitigen Lage (3) verbunden (9) ist,
und dass die luftundurchlässige Lage (3) unmittelbar vor der tatsächlichen Verwendung des Artikels abgerissen wird.

2. Bekleidungsartikel nach Anspruch 1, wobei das besagte Kühlmittel eine Größe hat, die mindestens 9% der Oberfläche des besagten Kerns auf der der besagten Oberlage zugekehrten Seite bzw. der Oberfläche des besagten Kerns auf der der besagten rückseitigen Lage zugekehrten Seite entspricht.

## Revendications

1. Article vestimentaire jetable absorbant pour fluides anatomiques (1) comportant une feuille de dessus perméable aux liquides (2), une feuille de support imperméable aux liquides (3), une partie centrale absorbant les fluides anatomiques (4) prise en sandwich entre ces deux feuilles et un élément refroidissant (7), dans lequel :
ledit élément refroidissant est une feuille gélatineuse à eau adaptée pour exercer une fonction de refroidissement quand ladite eau commence à s'évaporer, **caractérisé en ce que**
(i) ledit élément refroidissant (7) est intercalé entre ladite feuille de dessus (2) et ladite partie centrale (4), ou entre ladite partie centrale (4) et ladite feuille de support (3), ladite feuille de support étant imperméable à l'air, et ledit élément refroidissant est enfermé par une feuille imperméable à l'air (8) recouvrant une surface de ladite feuille de dessus (2) selon une orientation allant à l'opposé de ladite partie centrale et collée (9) de manière détachable sur cette surface et ladite feuille de support (3), ou
(ii) ledit élément refroidissant (7) est mis en oeuvre sur une surface extérieure (6) de ladite feuille de support (3) selon une orientation allant à l'opposé de ladite partie centrale (4), ladite feuille de support étant d'une nature imperméable à l'air, et d'une telle manière que ledit élément refroidissant (7) est enfermé par une feuille imperméable à l'air (8) préparée séparément de ladite feuille de support et collée (9) de manière détachable sur ladite surface extérieure (6) de ladite feuille de support (3) et ladite feuille de support (3) et la feuille imperméable à l'air est déchirée immédiatement avant que l'article ne soit utilisé à proprement dit.

2. Article vestimentaire selon la revendication 1, dans lequel ledit élément refroidissant a un prolongement correspondant à au moins 9 % de la surface de ladite partie centrale sur son côté selon une orientation vers ladite feuille de dessus ou la surface de ladite partie centrale sur son côté selon une orientation vers ladite feuille de support.
